# EUROPEAN PATENT SPECIFICATION

(11) **EP 4 068 152 B1**
(45) Date of publication and mention of the grant of the patent: **06.05.2026**
(21) Application number: 21166201.0
(22) Date of filing: 31.03.2021
(51) Int. Cl.: G01N 15/0227, C12M 1/36, G01N 15/06, G01N 15/1429, G01N 15/1433, G01N 15/10, G06N 3/084, G06N 20/10, G06T 7/00, G06V 20/69, G06N 3/0464, G06N 3/09, G01N 15/14

(54) **RAPID, AUTOMATED IMAGE-BASED VIRUS PLAQUE AND POTENCY ASSAY**
SCHNELLER AUTOMATISIERTER BILDBASIERTER VIRENPLAQUE- UND -KRAFTTEST
ANALYSE RAPIDE ET AUTOMATISÉE DE PLAQUE VIRALE ET DE PUISSANCE À BASE D'IMAGE

(43) Date of publication of application: 05.10.2022
(73) Proprietor: Sartorius BioAnalytical Instruments, Inc, Bohemia, NY 11716 (US)
(72) Inventor: OLSZOWY, Michael W., Ann Arbor, MI 48108 (US); REIF, Oscar-Werner, Ann Arbor, MI 48108 (US); TRYGG, Johan, Ann Arbor, MI 48108 (US); WALES, Richard, Ann Arbor, MI 48108 (US); SJÖGREN, Rickard, Ann Arbor, MI 48108 (US); EDLUND, Christoffer, Ann Arbor, MI 48108 (US)
(74) Representative: Novagraaf International SA

(56) References cited:
- ANDRIASYAN VARDAN ET AL: "Deep learning of virus infections reveals mechanics of lytic cells", BIORXIV, 9 October 2019 (2019-10-09), XP055839905, Retrieved from the Internet <URL:https://www.biorxiv.org/content/biorxiv/early/2019/10/09/798074.full.pdf> [retrieved on 20210910], DOI: 10.1101/798074

## Description

### Background

This disclosure is directed to methods and systems for performing cell-based, functional virus counting assays, and more particularly to methods and systems for allowing such assays to be completed in much less time than usual.

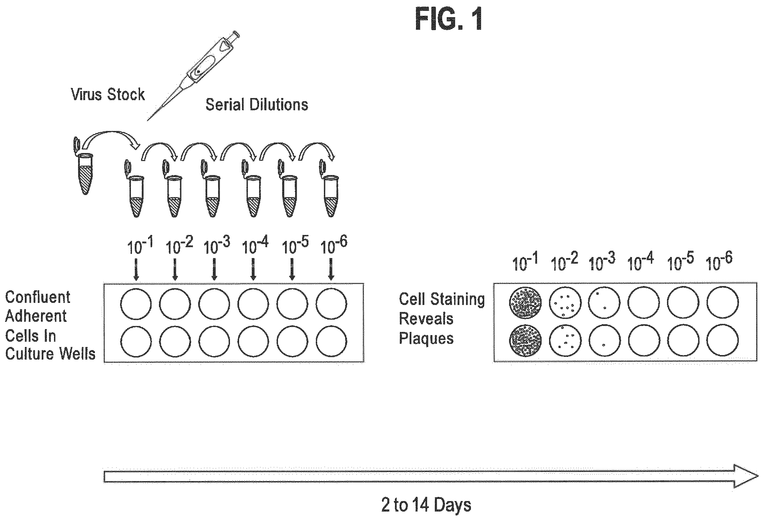

Functionality of viruses, such as active viruses, is currently measured in a variety of ways. The most widely used method is the standard plaque assay, which was first described in 1953. The assay measures virus function via the infection and lysis of target cells. The assay yields a plaque titer (concentration) indicative of the number of functional viruses, or plaque forming units, within a sample. The basic method is shown in Figure 1. First, cells are initially plated and grown to confluence. Then, virus samples of unknown concentration (titer) are serially diluted and added to the plates (often in the form of a petri-type dish or plate) containing the cells. Next, two to fourteen days later the cell monolayers are stained to reveal areas of lysis (plaques). Finally, the number of plaques is adjusted for dilution to determine virus titer of the original sample.

Other functional assays, such as the fifty-percent tissue culture infective dose (TCID₅₀), are derivative of the plaque assay. All involve incubation periods, virus with cells, of 2 to 12+ plus days depending upon the virus and cells used to measure functional infectivity.

There is wide disparity in the ratios of total virus particles, functional and non-functional, to plaque-forming units (PFUs) for different viruses, as indicated by Table 1. Infectivity titers or infectious particle counts and total particle counts are essential for full virus characterization. The particle to PFU ratio can vary across orders of magnitude.

**Table 1**

| Virus | | Particle-to-PFU ratio |
|---|---|---|
| *Adenoviridae* | | 20-100 |
| *Alphaviridae* | | 1-2 |
| | Semliki Forest virus | |
| *Herpesviridae* | | 50-200 |
| | Herpes simplex virus | |
| *Orthomyxoviridae* | | 20-50 |
| | Influenza virus | |
| *Papillomaviridae* | | 10,000 |
| | Papillomavirus | |
| *Picomaviridae* | | 30-1,000 |
| | Poliovirus | |
| *Polyomaviridae* | | |
| | Polyomavirus | 38-50 |
| | Simian virus 40 | 100-200 |
| *Poxviridae* | | 1-100 |
| *Reoviridae* | | 10 |
| | Reovirus | |

Source: http://www.virology.ws/2011/01/21/are-all-virus-particles-infectious/.

Therefore, it has become critically important to understand both the functional virus titer, via a plaque assay, as well as the total virus particle number in virus samples, both for commercial applications, such as vaccine development and manufacture, as well as for safety in gene therapy.

To address the need for total particle counts, more modern technologies, such as embodied in Sartorius' Virus Counter^{®} product, electron microscopy and a number of indirect methods, have been developed to provide total particle counts. For some of these technologies, these counts can be measured in as little as 30 minutes. Unfortunately, heretofore there are no rapid surrogates for the legacy virus plaque assay of Figure 1. It is highly desirable to have both total particle and infectious particle counts contemporaneously, or nearly so. This disclosure presents a rapid, automated image-based virus plaque and potency assay that provides a plaque assay titer, and in turn, an infectious particle count, in hours, rather than in days. Thus, this disclosure now makes it possible to obtain total particle and infectious particle counts essentially contemporaneously.

"Deep learning of virus infections reveals mechanics of lytic cells", Andriasyan et al., 2019, describes a deep learning approach to identify herpesvirus and adenovirus infections in the absence of virus-specific stainings. Procedures comprises staining of infected nuclei with DNA-dyes, fluorescence microscopy, and validation by virus-specific live-cell imaging. Deep learning of multi-round infection phenotypes identified hallmarks of adenovirus-infected cell nuclei. At an accuracy of >95%, the procedure predicts two distinct infection outcomes 20 hours prior to lysis, nonlytic (nonspreading) and lytic (spreading) infections.

### Summary

In one aspect, described herein is a method for training a machine learning model to predict virus titer from an image, or sequence of images, of a cell culture containing a virus population. In this document, the term "machine learning model" refers to a computational system that has used optimization algorithms to learn and perform a task based on previous examples of desired input-output pairs. The trained machine learning model allows a prediction of virus titer to be made much earlier than in the standard virus plaque assay, for example in 6 or 8 hours (or possibly less) after initial inoculation of the cell culture with the virus sample, as compared to many days in the prior art. The method of training the machine learning model can include the steps of: (1) obtaining a training set in a form of a plurality of images of virus-treated cell cultures from a plurality of experiments at one or more time points from a start time t₀ to a final time t_{final}, (2) for each experiment, recording at least one numeric virus titer readout of the virus-treated cell culture at time t_{final}, (hereafter "ground truth"), (3) processing all the images in the training set to acquire a numeric representation of each image, and (4) training one or more machine learning models to make a prediction of a final virus titer on the training set numeric representations.

Also described herein is an application of the trained one or more machine learning models as a method of predicting a virus titer of a cell culture to which a virus sample of unknown titer has been added. In this "application" phase, the method can include the steps of: a) obtaining a time sequence of images of the cell culture, b) supplying a numeric representation of the time sequence of images obtained in step a) to one or more machine learning models trained in accordance with the previous paragraph, and c) making a prediction with the one or more trained machine learning models of the virus titer.

In another aspect, an analytical instrument is provided that is configured to hold one or more plates containing a cell culture and a virus sample. The instrument includes an integrated imaging system. The instrument is configured with a machine learning model trained to make a prediction of a virus titer in the cell culture from one or more images in a time sequence of images of the cell culture obtained by the imaging system, wherein the prediction is made before the viral infection of the cell culture has proceeded to term. For example, the prediction can be made at 4, 6, 10 or 15 hours, as an example, after initiation of the viral infection, instead of several days.

In one further aspect, this analytical instrument can be further configured with a processing unit executing a training module which enables the user of the instrument to conduct a training procedure to create a new trained machine learning model to make a virus titer prediction. This training module provides set-up instructions for facilitating a user of the instrument conducting a training method with the instrument. This training method can include the steps of: (1) obtaining a training set in the form of a plurality of images of virus-treated cell cultures from a plurality of experiments at a set of time points from a start time t₀ to a final time t_{final}, (2) for each experiment, recording at least one numeric virus titer readout of the virus-treated cell culture at time t_{final}, (3) processing all of the images in the training set to acquire a numeric representation of each image, and (4) training one or more machine learning models to make a prediction of a final virus titer on the numeric representations in the training set, wherein the training comprises minimizing an error between the model prediction of a final virus titer and a ground truth.

Several different methods can be used for the processing step (3) in the model training. In one embodiment, the processing step (3) involves passing the images through a convolutional neural network (CNN) to thereby acquire an intermediate data representation of the images. In another embodiment, the processing step (3) takes the form of sub-steps a)-c): a) segmenting individual cells from the image, b) calculating a cell-by-cell numeric description of each cell, and c) aggregating the numeric descriptions over all cells.

### Brief Description of the Drawings

Figure 1 is an illustration of a prior art virus titer assay.
Figure 2 is an illustration of a method for training a machine learning model to make a prediction of virus titer from a time sequence of images between an initial time t₀ and a final time t_{final} and to provide a numeric virus titer readout of the virus-treated cell culture at time t_{final}, hereafter "ground truth"; Figure 2 also shows the application of the trained model to a new model input in the form of one or more images. The trained model produces an output in the form of a predicted virus titer.
Figure 3 is an illustration of one possible manner of reporting the model output to a user.
Figure 4 is an illustration of one possible example of an analytical instrument which can be used to implement the model training and model application phases of this disclosure.
Figure 5 is an illustration of the fluorescence imaging system in the analytical instrument of Figure 4.
Figure 6 is a flowchart diagram illustrating a training phase when a single machine learning model is trained from numeric representations of images from multiple time points.
Figure 7 is a flowchart diagram illustrating a training phase when a separate machine learning model is trained per time point.
Figure 8 is a flowchart diagram illustrating a model application phase which uses a single machine learning model that has been trained to use numeric representations from multiple time points during a training phase.
Figure 9 is a flowchart diagram illustrating a model application phase which uses a single machine learning model that has been trained to use numeric representations per time point during a training phase.
Figure 10 is a flowchart diagram illustrating a model training phase in which the loss or prediction error between the machine learning model prediction and the ground truth is minimized.
Figure 11 is an illustration of a sequence of steps performed in one alternative embodiment of the processing of images step (3) of the model training procedure.
Figure 12 is an illustration of a display on a workstation which is associated with the analytical instrument of Figure 4 showing a set-up menu which allows the user to run a virus titer assay using the instrument.

### Detailed Description

### Overview

This disclosure provides a method of predicting a virus titer readout at experiment end time, or *t*_{final}, at a time point t which is less than (or earlier than) *t*_{final}. The phrase "experiment end time," or *t*_{final}, refers to the time when a virus titer assay has been allowed to run to completion, or equivalently, proceeded to term, i.e., when visible plaques have formed, and is typically 2 days or more depending on the virus or family of viruses in question, the cell type the virus is allowed to grow in, and other factors which are known in the art. The disclosed methods allow this predicted virus titer readout to be made long before the usual experiment end time, for example in 6 - 8 hours, or possibly even earlier, for some virus titer assays, instead of a matter of days.

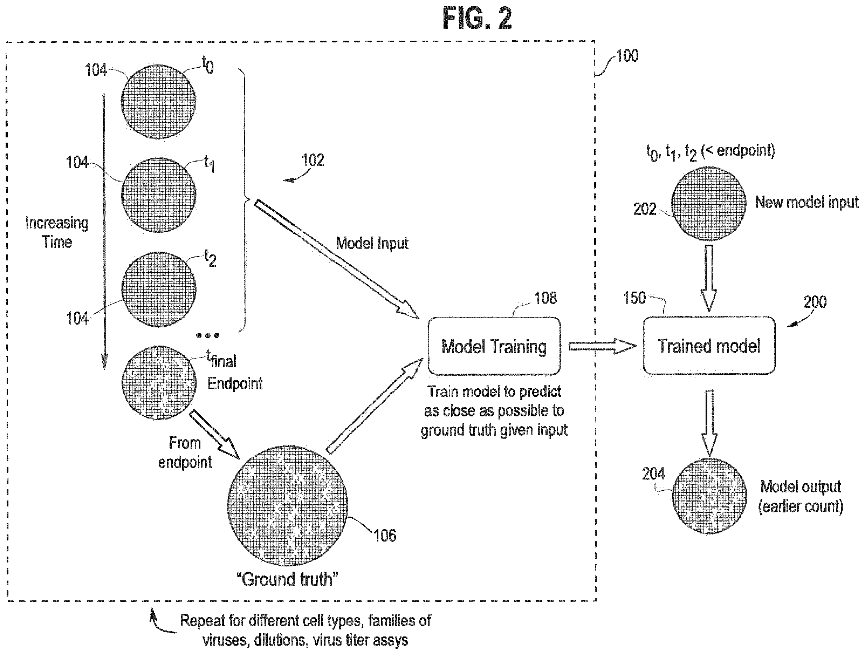

The method involves the training and use of a one or more machine learning models that are used to make this prediction. This disclosure therefore involves two different aspects, shown in Figure 2, namely a machine learning model training phase 100 in which a trained machine learning model 150 is developed, and a machine learning model application phase 200 in which the trained machine learning model 150 is applied to a new model input 202 and a model output 204 in the form of a predicted virus titer is generated by the trained machine learning model 150.

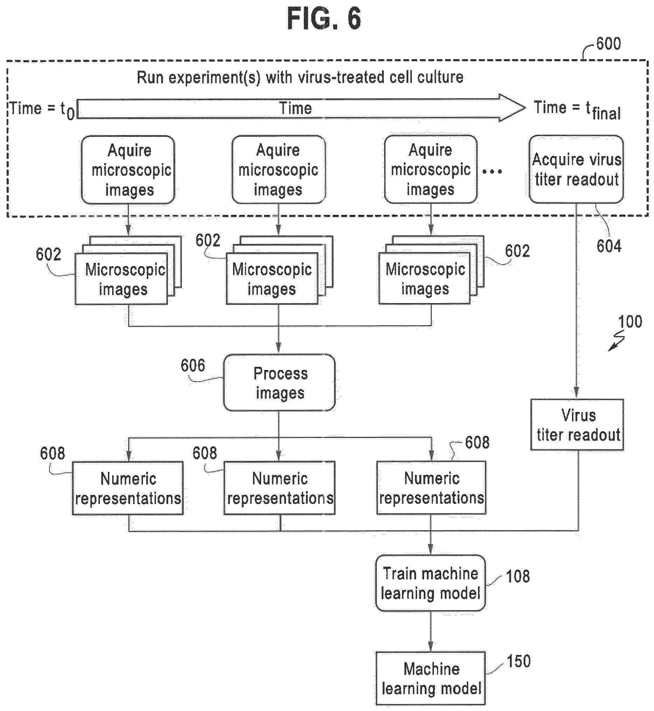

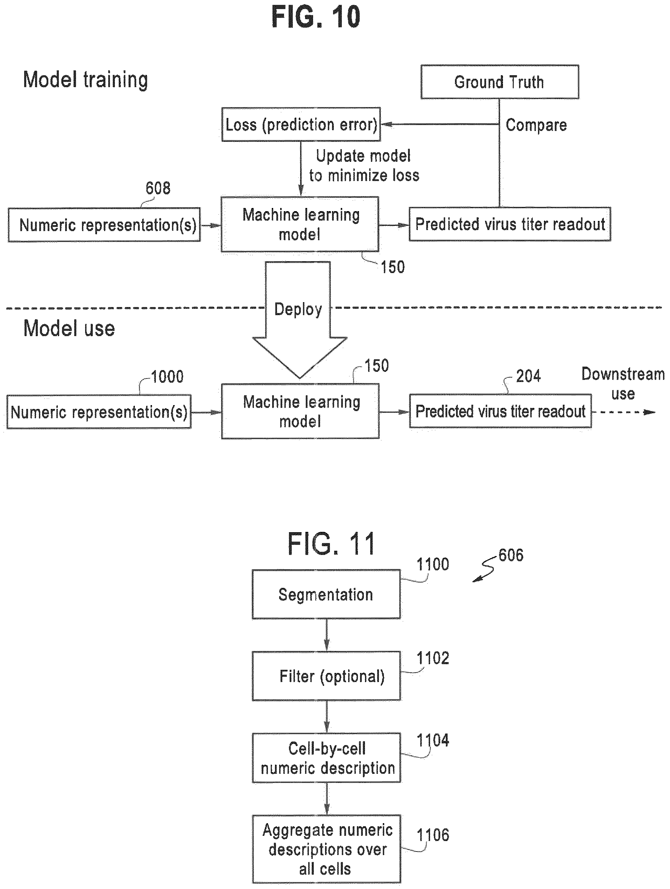

The model training phase 100 can include several steps. First, in step (1) a training set 102 is obtained in the form of a plurality of sets of images, typically microscopic images 104 of virus-treated cell cultures from a plurality of experiments at one or more time points between a start time t₀ to a final time t_{final}. The time points t*₁*, t*₂*, ... could be periodic, such as every 30 or 60 minutes, for example. In step (2), for each experiment, a recording is made of at least one numeric virus titer readout of the virus-treated cell culture at time t_{final}, hereafter "ground truth" 106. While Figure 2 shows this ground truth as an image, it can be represented as a number, for example, number of infective or infectious particles, or number of infectious particles per unit volume, or other metric known in the art to represent a virus titer (concentration). At step (3), a processing of all the microscopic images in the training set 102 is performed to acquire a numeric representation of each microscopic image. This step is not shown in Figure 2 but is shown in the discussion of the embodiments of Figures 6-11 and will be described in detail below. In step (4), a training of one or more machine learning models 108 is performed to make a prediction of a final virus titer on the training set numeric representations. The training involves minimizing the loss or prediction error between the model prediction of a final virus titer and the ground truth, as described in Figure 10 and discussed in detail below.

The model application phase 200 of Figure 2 is where the trained machine learning model 150 is used to make a prediction of virus titer. In particular, described is a method for predicting a virus titer of a cell culture to which a virus sample of unknown titer has been added, which can include the steps of: a) obtaining a time sequence of microscopic images of the cell culture 202, for example at every thirty minutes after inoculation of the cell culture with the virus occurs; b) supplying a numeric representation of the time sequence of microscopic images obtained in step a) to one or more machine learning models trained in accordance with the model training phase 100, and c) making a prediction with the one or more trained machine learning models of the virus titer, shown as model output 204. The numeric representation step on the model input is performed using the same method as described below for generating numeric representations of the training set 102 in the form of microscopic images 104. The model output 204 is shown in Figure 2 as an image but it can be represented as a number, or other known metric known in the art to represent a virus titer (concentration).

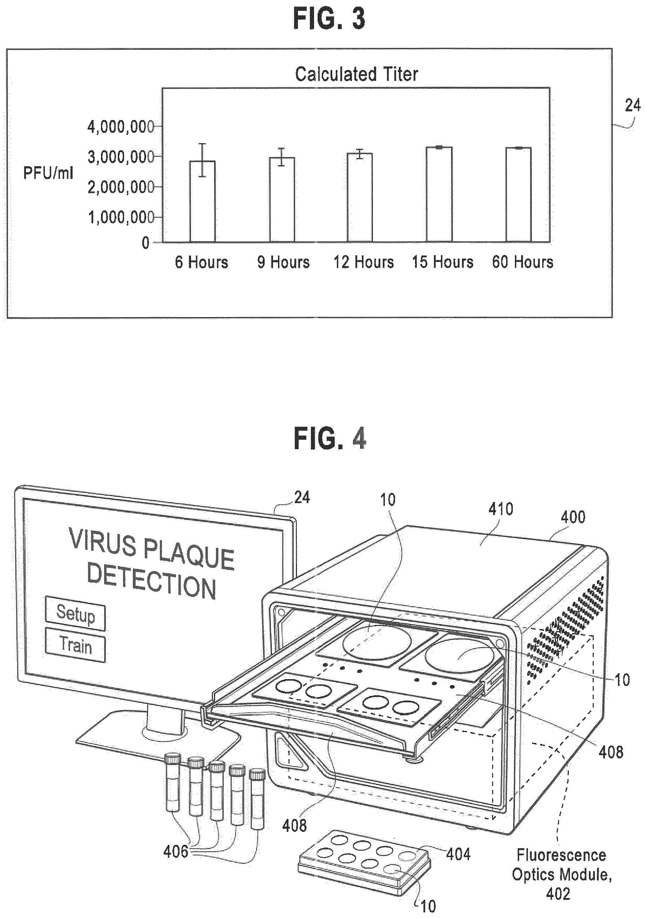

An example of the model output 204 is shown in Figure 3. The model output is shown in the form of a plot of "calculated titer" (or, equivalently, predicted titer) as a function of time over several time periods after initial inoculation of the cell culture with the virus, presented on a display of a workstation 24 of an instrument which performs the model application process or phase 200 of Figure 2. In this particular (hypothetical) example of Figure 3, the model output 204 includes a scale on the left hand and plots of predicted virus titer at 6 hours, 9 hours, 12 hours, 15 hours and 60 hours, with error bars indicating the uncertainty of the prediction at each time point, such as 2,800,000 +/- 500,000 PFU (plaque forming units)/mL at 6 hours, 2,900,000 +/- 300,000 PFU/mL at 9 hours, etc. Note that these predictions can be made with ever increasing precision starting approximately at 6 hours in this example, with high precision in the virus titer at 12 hours and even greater precision at 15 hours.

Referring back to Figure 2, the model training process or phase 100 can be repeated many times in order to acquire a suite of trained machine learning models. This is because different viruses or families of viruses will exhibit different rates of infection and cell lysis in particular cell types. Therefore, to generate models that are accurately predictive of virus titer across many different cell types and virus families, the training procedure could be conducted for each family of virus and for each of the commonly used cell lines in viral research. Additionally, it may be advantageous to develop machine learning models that are predictive at different points in time, for example at 6 hours, 12 hours, 15 hours, etc., rather than a single machine learning model trained from a set of images generated over the entire period between t₀ and t_{final}, as will be explained below. Additionally, it may be desirable to repeat the model training process or phase 100 for different dilutions of the virus (see the left-hand side of Figure 1).

### Example Analytical Instrument

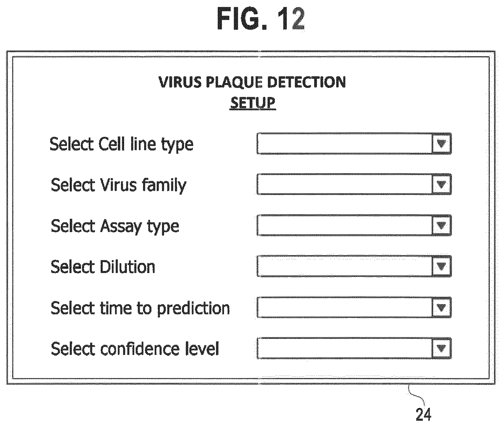

The method of this disclosure can be performed in any suitable machine or instrument which includes a mechanism for obtaining images of the cell culture with added viral sample at different points in time. Preferably, such images are microscopic images. One example of such an instrument is shown in Figure 4, and the following description is offered by way of example and not limitation. The instrument 400 in the illustrated embodiment is the Incucyte^{®} live-cell imaging system of the Assignee. The instrument 400 is adapted and configured for obtaining images from live cells or cell cultures having different possible formats, including microwell plates, cell culture plates, and the like. The instrument 400 includes a housing 410, the entirety of which could be placed inside a temperature and humidity-controlled incubator, not shown, during use. The instrument 400 is adapted to receive cell culture plates 404 which include one or more holding wells 10, each of which receives a cell culture and virus sample of unknown concentration of plaque forming units. Further, the instrument 400 is compatible for use with a reagent kit which can include an optional set of fluorescence and/or immunohistochemical reagents 406, one or more of which are added to each of the wells 10 so as to enable fluorescence or immunohistochemical measurements from the cell line sample to be obtained. The system includes an associated workstation 24 which implements a machine learning model training process and/or application process (Figure 2, procedures or phases 100 and/or 200) and display features to enable the researcher to see the results of virus titer experiments conducted on the sample. In Figure 4, the display of the workstation shows the user has entered a "virus plaque detection" application which allows the user to select a "setup" menu (see Figure 12) and enter the parameters and information needed to conduct a model application process or phase (Figure 2, 200) or a "train" menu which would allow the user to set up the procedures for conducting a model training process or phase (Figure 2, 100).

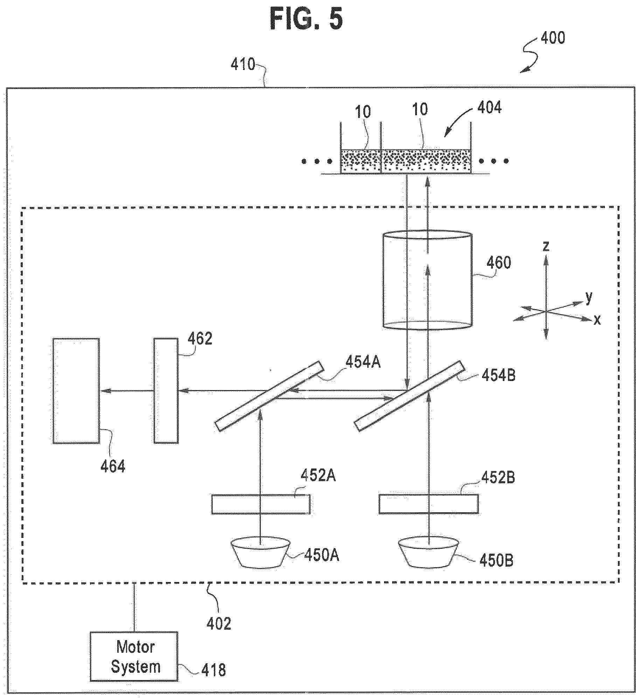

The instrument 400 includes a tray 408 which slides out of the system and allows the culture plate 404 to be placed onto the tray 408 and then retracts and closes so as to place the culture plate 404 within the interior of the housing 410. The culture plate 404 remains stationary within the housing while a fluorescence optics module 402 (see Figure 5) moves relative to the plate 404 and obtains a series of fluorescence images over the course of an experiment. In a variation of this embodiment, the images that are acquired could be brightfield, non-fluorescent images.

Figure 5 is a more detailed optical diagram of the fluorescence optics module 402 of Figure 4. Further details on the fluorescence optics module 402 shown in Figure 5 can be found in U.S. patent application of Brad Neagle et al., serial no. 16/854,756 filed April 21, 2020, entitled "Optical module with three or more color fluorescent light sources and methods for use thereof", assigned to the assignee of this invention. The details of the optics module 402 of Figure 5 are not particularly important and can vary widely from what is shown in the Figure, and as such Figure 5 is offered by way of example and not limitation.

The module 402 includes LED excitation light sources 450A and 450B which emit light at different wavelengths, such as 453-486 nm and 546-568 nm, respectively. The optics module 402 could be configured with a third LED excitation light source (not shown) which emits light at a third wavelength, such as 648-674 nm, or even a fourth LED excitation source at a fourth different wavelength. The light from the LEDs 450A and 450B passes through narrow bandpass filters 452A and 452B, respectively, which pass light at particular wavelengths that are designed to excite fluorophores in the cell culture and virus medium. The light passing through the filter 452A reflects off a dichroic 454A and reflects off dichroic mirror 454B and is directed to an objective lens 460, e.g., a 20X magnifying lens. Light from LED 450B also passes through the filter 452B and also passes through the dichroic mirror 454B and is directed to the objective lens 460. The excitation light passing through the lens 460 then impinges on the bottom of the plate 10 and passes into the medium 404. In turn, emissions from the fluorophores in the sample pass through the lens 460, reflect off the mirror 454B, pass through the dichroic 454A, and pass through a narrow band emission filter 462 (filtering out non-fluorescence light) and impinge on a digital camera 464, which may take the form of a charge coupled device (CCD) or other type of camera currently known in the art and used in fluorescence microscopy. A motor system 418 then operates to move the entire optics module 402 in the X, Y and optionally Z directions while the light source 450A or 450B is in an ON state. It will be appreciated that normally only one optical channel is activated at a time, for example the LED 450A is turned on and image is captured, then LED 450A is turned off and LED 450B is activated, and a second image is captured.

It will be appreciated that the objective lens 460 can be mounted to a turret which can be rotated about a vertical axis such that a second objective lens of different magnification is placed into the optical path to obtain a second image at a different magnification. Furthermore, the motor system 418 can be configured such that it moves in the X and Y directions below the plate 404 such that the optical path of the fluorescence optics module 402 and the objective lens 460 is placed directly below each of cell cultures in the various wells 10 of the plate 404.

The details of the motor system 418 for the fluorescence optics module 402 can vary widely and are known to persons skilled in the art.

The use of fluorescence and filters in Figure 5 is optional and in one embodiment brightfield images are acquired from broad spectrum illumination sources and without the use of excitation and emission filters.

In one embodiment, the virus sample is supplied to a separate instrument, such as the Sartorius Virus Counter^{®}, in order to acquire a total particle count, where that separate instrument can operate in parallel with the virus plaque assay in the instrument of Figures 4 and 5 in an application phase to acquire a plaque assay titer essentially contemporaneously with the total particle count.

### Example Embodiments

This section will describe numerous possible embodiments for the model training and model application phases, respectively, in conjunction with Figures 2 and 6-11.

As explained previously, there is a model training (or setup) phase 100 of Figure 2, and application phase 200 of Figure 2. The model training phase 100 can essentially be a 4-step process: (1) Acquire a training set 102 of images of the virus-treated cells, e.g., in a cell culture medium held within a well of a culture plate; repeat as a series of experiments. (2) Acquire and record at least one numeric virus titer readout for the cell culture medium imaged in step 1 at time t_{final}, or "ground truth" 106, in each experiment. (3) Process the images in the training set 102 to acquire a numeric representation of each microscopic image. (4) Train one or more machine learning models 108, e.g., a regression model, to predict the final virus titer based on the numeric representations from earlier time points. In this training step, a minimization of the loss or error between the predicted final virus titer and the ground truth is performed. Once this model (or set of models) is trained they are stored and then used during the model application phase 200. The model application phase 200 consists of (1) acquiring a time sequence of images of a cell culture inoculated with a virus of unknown titer, for which an early prediction of virus titer is desired, (2) processing the images in the same way as discussed above with respect to step 3 of the model training phase 100 into a numeric representation, and (3) applying the trained machine learning model to obtain a predicted virus titer.

As noted earlier, it is possible and preferable in some embodiments to go through or repeat the training phase to train models for commonly used cell types. Such models can then be shipped or provided as integrated parts of software modules to users of analytical instruments such as the one described above in Figures 4 and 5. The customer/user then only needs to go through the application phase. However, there may be situations where the customer/user of the instrument desires to perform virus titer experiments on uncommon cell types which are too dissimilar to the ones that models have been developed on using the process of Figure 2. In this case, the customer/user performs the model training process of Figure 2. The instrument supports this embodiment by offering the model training procedure as a software package that basically guides the user to implements the process or phase 100 of Figure 2, e.g., by entering the "train" module shown in the display of the workstation of Figure 4.

### Nomenclature

In the following discussion, certain meanings are ascribed to the terms used herein:
"Artificial neural network" (ANN) refers to type of machine learning model, consisting of multiple layers of nonlinear mathematical transformations with model parameters that are learned using optimization algorithms.

"Convolutional Neural Network" (CNN), refers to a type of ANN that is commonly used for processing data with spatial correlations, such as pixels forming shapes and objects in images.

"Activations" refers to an intermediate representation of input data as passed through layers of an ANN.

An example of the 4-step model training phase or process 100 and the 3-step application phase 200 shown in Figure 2 and described above will now be described in conjunction with Figure 6.

### Model Training (or Setup) Phase Steps (Figure 2, 100 and Figure 6)

Step 1. Acquire a training set in the form of one or more images, preferably microscopic images (602, Figure 6), of a virus-treated cell culture from a plurality of experiments 600 at one or more time points from start time t₀ until t_{final}. The same time point(s) are preferably used for all experiments. The time points could be spaced evenly or unevenly and could be periodic with a period of 60 minutes or less, such as every 30 minutes. One experiment 600 may comprise a cell culture growing in a well in a culture plate and the plurality of experiments may comprise multiple wells of cell culture treated with different virus concentrations. This set of images 602 will hereon be denoted as the training set. Depending on the field of view of the camera obtaining the images, the images may be combined or stitched together to create a wide field of view image of the entire cell culture. Alternatively, individual small field of view images could be acquired and processed without generating a composite or combined overall image.

The microscopic images 602 may include label-free light microscopic images, such as brightfield images or phase contrast images.

The microscopic images 602 comprise fluorescence images of the cell culture labelled with a fluorescent marker of interest. In this embodiment, the fluorescent marker is a fluorescent antibody binding to virus-specific protein epitopes, expressed on the surface or interior of virus-infected cells.

The microscopic images 602 may also include immunohistochemistry images, brightfield and phase, of the cell culture labeled as the result of the enzymatic action of a chromogenic detection system. This chromogenic marker may be an enzyme-linked direct primary antibody binding to virus-specific protein epitopes, expressed on the surface or interior of virus-infected cells. Alternatively, the chromogenic marker may be a secondary antibody with affinity for a first antibody, the latter specific for virus-specific protein epitopes, expressed on the surface or interior of virus-infected cells. As another possibility, the chromogenic detection system may be a combination of the horseradish peroxidase (HRP) enzyme conjugated to the primary or secondary antibody and the insoluble product made as a result of the action of HRP on 3,3'-diaminobenzidine tetrahydrochloride (DAB). As a still further possibility, the chromogenic detection system may be one of a plurality of other pairs of immunohistochemistry detection systems. See, for example, https://www.abcam.com/kits/substrates-and-chromogens-for-inc.

The microscopic images 602 may be pairs of label-free light microscopic images and fluorescence images labelled with a fluorescent marker as explained above.

Step 2. For each experiment imaged in the training set, record at least one numeric virus titer readout 604 of the virus-treated cell cultures at t_{final}. The virus titer readout may be recorded manually by visual inspection, or automatically using a computational algorithm to process the image(s) at t_{final}. These virus titer readouts will hereon be denoted as the ground truth target, or simply "ground truth."

The virus titer readout may be the readout from a plaque assay. In particular, the plaque assay readout may be the number of individual plaques (i.e., a standard readout).

Alternatively, the plaque assay readout may also be the area covered by plaque. (option a)

As a variation, the virus titer may be the readout from a plaque assay wherein the plaque assay readout is acquired automatically by using an image segmentation algorithm to segment cell mass from background and plaques as holes of absent cell mass forming during the duration of the experiment. (option b)

As another variation, the virus titer readout may be the readout from a Tissue Culture Infective Dose 50% Assay (TCID₅₀). (option c)

As still another variation, the virus titer readout may by the readout from a focus forming assay (FFA). (option d)

As still another possibility, the virus titer readout may be combination of the above options, for example options a or b and option c; or options a or b and option d.

Step 3. Process all microscopic images in the training set to acquire a numeric representation 608 of each image (pair, in case fluorescent images are used), step 606 in Figure 6.

The processing 606 may consist of passing the whole images through a CNN to acquire a set of CNN activations per image.

As an alternative, the processing 606 may also or alternatively consist of the process steps shown in Figure 11: step 1100 - segmentation, or segmenting each individual cell from the image, step 1102 - an optional filtering step, step 1104 - calculating a cell-by-cell numeric description of each cell, and step 1106 - aggregating the numeric descriptions over all cells.

The segmentation (step 1100) may be performed by various possible techniques, such as:
Label-free cell segmentation using traditional computer vision algorithms, label-free cell segmentation using a CNN for cell instance segmentation, or thresholding the membrane marker fluorescent image as given by the procedure of step 1 of using a cell membrane marker.

The cell-by-cell numeric description step 1104 may be calculated in a number of different manners. For example, one can use either of the following methods:
1. Extracting morphological features using feature extraction as in the process of calculating a human defined set of feature descriptors based on cell area, eccentricity, pointiness, minor/major-axis, granularity, etc.
2. Extracting morphological features by feeding the segmented sub-images of cells to a CNN to extract a machine learning-defined set of feature descriptors.
3. Extracting fluorescence levels as defined by the intra-cell sum of fluorescent pixels based on fluorescent image (where the cell culture is labeled with a fluorescence marker of interest, as explained in step 1).

The aggregation step 1106 may be performed in several possible methods. For example, it can be performed by calculating the feature-wise average over all cells in an image, calculating the ratio between different types of cells as defined based on the cell-by-cell numeric descriptions, or performing dimension reduction and calculating the probability distribution of the reduced dimension-space over all images and then aggregating each image as the distribution over the reduced dimension according to the probability distribution defined based on all images. This latter method refers to the single cell-shape distribution analysis as described in EP Patent Application 20290050.2 filed June 12, 2020. Alternatively, the aggregation step can be performed by feeding the cell-wise feature descriptions to a set-invariant neural network (such as Deep Sets, see Zaheer, Manzil, et al. "Deep sets", Advances in neural information processing systems 30 (NIPS 2017)).

As noted in Figure 11, there is an optional filtering step 1102. In particular, steps 1104 and 1106 of Figure 4 can be performed using only virus-infected cells as filtered out by first thresholding the intra-cell sum of fluorescent pixels based on the optional fluorescent image (obtained in Step 1 in which fluorescent images are obtained), or using a machine learning-model trained to perform label-free classification of cells as virus-infected or not.

As another example of the filtering step 1102 of Figure 11, the processing steps 1106 and 1106 may be performed by filtering out dead cells. Such dead cells could be identified by thresholding the intra-cell sum of fluorescent pixels based on the optional fluorescent image in which a cell death marker is used in Step 1, or by using a machine learning-model trained to perform label-free classification of cells as dead cells or not dead cells.

As another example of the filtering step 1102 of Figure 11, a combination of filtering out non-virus infected cells (as explained above) and filtering out dead cells (as explained above) may be performed in order to filter out dead cells that did not die due to virus infection.

Step 4. Train one or more machine learning models (step 108) on the training set numeric representations (608) to minimize the difference (or equivalently, error or loss) between the model prediction of the virus titer (predicted plaque assay) at t_{final} and the ground truth, resulting in a trained machine learning model 150. See Figure 10.

The model 150 may take a variety of forms. For example, it could be a linear model, such as a partial least squares regression model. Alternatively, it could be a non-linear model, such as an ANN. As another example, the model 150 could be a probability distribution over the plaque assay readout, such as Gaussian process regression. See Rasmussen, Carl Edward, "Gaussian processes in machine learning." Summer School on Machine Learning. Springer, Berlin, Heidelberg (2003). As another example, the model 150 could be a dynamic model, such as a neural ordinary differential equation model. See e.g., Chen, Ricky TQ, et al., "Neural ordinary differential equations," Advances in neural information processing systems 31 (NeurIPS 2018).

The model 150 may be trained by iteratively adjusting the model parameters to minimize the error of the predicted plaque assay readout compared to the ground truth. This error could be given as a mean-squared error, a mean-absolute error, or as a piece-wise absolute, piece-wise squared error, also known as "Huber loss." See Huber, Peter J., "Robust estimation of a location parameter", Breakthroughs in statistics. Springer, New York, NY, 1992, pp. 492-518.

Note: When ANN models are used in two or more consecutive steps, they may optionally be connected and the virus titer prediction loss is back-propagated through the multiple sub-ANNs to optimize them jointly.

### Application Phase Steps (Figure 2, 200)

During the application phase, one or more experiments are run with virus-treated cell culture(s) for which the virus titer readout will be predicted earlier in time based on the model trained during the model training phase. For a given experiment, the final virus titer readout count is predicted at a time point t < t_{final} by:
1. Acquiring one or more microscopic images of the experiment cell culture (Figure 2, 202) until time point t. The image(s) are preferably acquired using the same image acquisition protocol as step 1 of the model training phase 100.
2. Processing the acquired image(s) into numerical representation(s) (Figure 10, 1000) using the same image processing protocol as step 3 of the model training phase 100 described above.
3. Predicting the final virus titer 204 by applying the trained machine learning model 150 to the numerical representation(s) 1000, as shown in Figure 10.

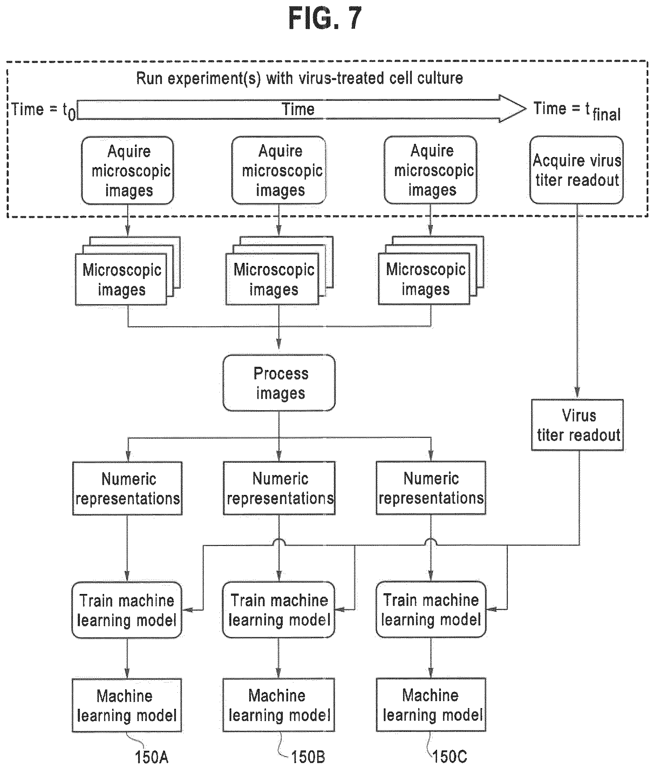

Figure 7 is a flowchart diagram illustrating a model training phase when a separate machine learning model is trained per time point. There are three such models 150A, 150B, 150C shown in the Figure, but it will be appreciated that there may be more, such as possibly 10 or 20 or more of such models, for example when there are 10 or 20 time points at which images are acquired, for example every 30, 45 or 60 minutes during each experiment performed during the training phase. The other steps in Figure 7 are the same as explained above in conjunction with Figure 6. The embodiment of Figure 7 could be used in the application phase (Figure 2, 200) where, for example, images are acquired every 30 minutes over a six hour period, and the 12^{th} trained model 150, trained at the six hour time period during model training, is then used to make a prediction of the final virus titer six hours after the experiment commenced. Similarly, as the images are collected at the application phase at each 30 or 60 minute interval, the numerical representation of each image is then supplied to the associated trained machine learning model at each interval, models 150A (30 minutes), 150B (60 minutes), 150C (ninety minutes), 150D (120 minutes), and so on and so forth, and each model makes a prediction and the results are generated and displayed for the user for example as shown in Figure 3, along with the error bars or uncertainty in the prediction.

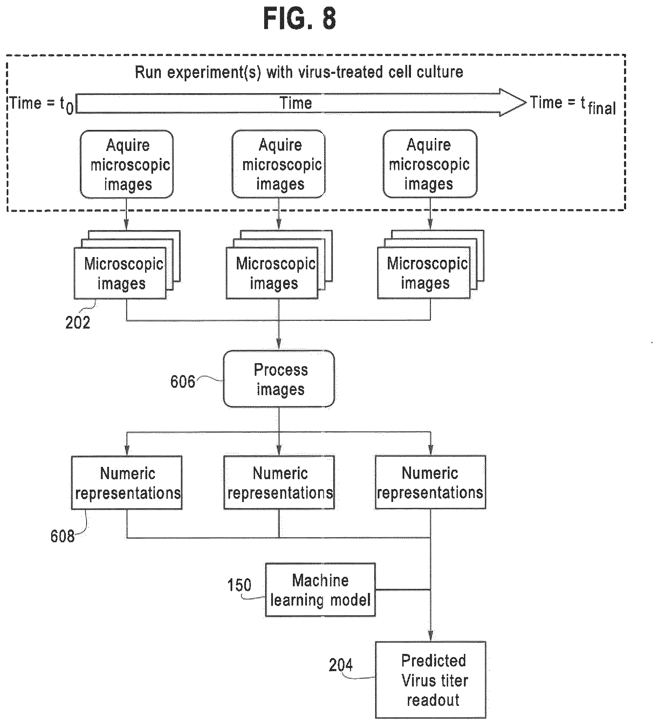

Figure 8 is a flowchart diagram illustrating a model application phase (Figure 2, 200) in the situation where a single machine learning model 150 was trained to use numeric representations 608 from images 202 obtained at multiple time points, in accordance with the procedure described in Figure 6. The process images module 606 creates numeric representations of the images as explained above, and the numeric representations 608 are input to the trained model 150 and a prediction of virus titer is made as indicated at 204.

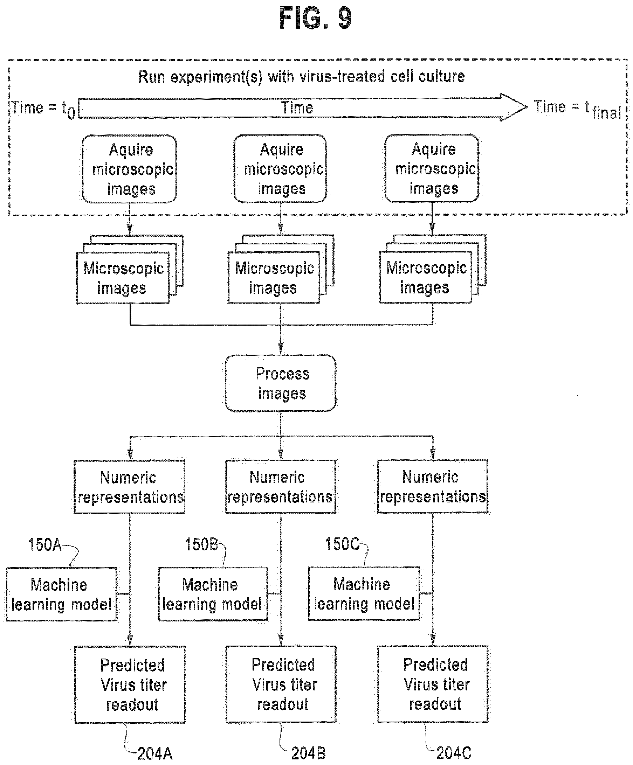

Figure 9 is a flowchart diagram illustrating a model application phase in the situation where a single machine learning model was trained per time point during the model training phase, in accordance with the training process shown in Figure 7. The training process results in multiple trained machine learning models 150A, 150B and 150C (and optionally additional models, such as for example 20 additional such models, not shown). The numeric representation of the images at each time point are then supplied to the associated machine learning models for that time point and each model 150A, 150B, 150C, ... makes a prediction of the virus titer readout 204A, 204B, 204C, ... respectively.

Referring now to Figures 4 and 12, the workstation associated with the analytical instrument 400 can include a display 24 which provides for the tools or interfaces that are needed for the user of the instrument to run the virus titer assay described herein. While the details of the display 24 can vary widely, it will typically include features which allow the user to input the information necessary in order for the software of the instrument to select the appropriate machine learning model(s) stored in memory to make predictions as the cell culture and virus set to be imaged within the instrument. For example, as shown in Figure 12, the user is given a menu to select things such as:
the type of cell line in their experiment,
the type of virus family being inoculated into the cell line,
the assay type (e.g., plaque forming unit count per unit volume, TCID ₅₀, both, other, etc.)
the dilution level in the cell plate,
the time or time periods after the start of processing that the prediction is desired (e.g. 4 hours, 6 hours, 15 hours, every 30 minutes or every hour, etc.).

Optionally, the menu can include a confidence level feature in which the user can program the application such that only predictions within a certain confidence interval or error limit are reported, and predictions with larger uncertainty are not reported. The interface shown in Figure 12 is just one possible example and is offered by way of example and not limitation, and the details of the design of the interface and the menu options can vary widely from what is shown in Figure 12.

Additionally, the menu can include an option to enter into a training mode whereby the user sets up the experimental design to train new, additional machine learning models to predict virus titer. For example, the display of the instrument could include a "TRAIN" icon (see Figure 4) which when activated allows the user to enter the experimental parameters to conduct model training as explained above.

The trained machine learning model could be implemented in a processing unit of the instrument 400 of Figure 4, or alternatively on a remote computing platform connected to the instrument. The instrument 400 of Figure 4 further could optionally include a model training module which permits the user of the instrument to perform the model training process of this disclosure using the instrument of Figure 4.

### Further considerations

As explained above, an embodiment is described in which an image or a sequence images is taken and segmentation algorithms are used to identify individual cells, see Figure 11, step 1100. These segmentation algorithms may be based on a convolutional neural network (CNN) performing instance segmentation, or they may be based on the existing cell-by-cell type of algorithms. Each individual cell can then be described using a multi-attribute representation quantifying phenotype, shape and texture in one or many different ways. In particular, the images can be examined on a pixel-by-pixel level, providing additional information as to patterns and texture between pixels in fine detail. Based on this generated feature rich dataset of cell related descriptors and/or other related metadata, multivariate data analysis can then be used to detect cytopathic effects specifically related to viral activity. Based on the observed cytopathic effect, machine learning models can then be trained to predict the viral activity. The machine learning may use a single time-point as input for prediction or multiple time-points leading up to the current time. This embodiment may be modified by omitting the use of cell segmentation algorithms and use patch-wise descriptions of the image(s) instead. Due to the nature of the cell carpet, cell subpopulations may be approximated by dividing the image(s) into a regular grid, with each grid element described based on shape and texture parameters and predict viral activity in the same way as described.

Furthermore, because the methods are based in image-based trained models, the methods permit determination of a degree of cytopathic effects by predicting information such as how large, how many, and specific location of the plaques. Moreover, just because cytopathic effects are present in a sample, that this does not necessarily equate to plaque assay formation. The plaque assay of this disclosure enables a prediction of whether the cytopathic effects will develop into plaques. It is also not obvious or expected that all cells exhibiting cytopathic effects will lead to or result in plaque formation. Certain environmental conditions have to present for plaque formation to occur. The live-cell, unperturbed, imaging capabilities of an incubator-based microscope, such as shown in Figures 4 and 5, which maintains a certain pH and physiological temperature of the cell culture, allows for the monitoring and observation of the structural changes in the host cell caused by viral invasion and thus plaque formation.

The various embodiments of this disclosure may be complemented by in-silico labelling. In-silico labelling means that a machine learning or deep learning model has been trained to predict the corresponding fluorescent image of a fluorescent label of interest. Given a dataset of virus-treated cell cultures that is labelled with a label indicating viral activity, degree of infection, etc., a machine learning or deep learning model may be trained to predict the label from a corresponding light microscopy image. This trained model may then be applied to further image sets to predict a corresponding label in a label-free fashion. The predicted label can then be used as auxiliary input in the plaque prediction model or used as additional description of cells/grid elements in which predictions are assigned to particular cells in a grid.

Multivariate data analysis may also detect other phenotypic effects, either time-related or at a single time point, such as cell detachment and rounding during normal replication that are not directly related to viral activity and used as part of the prediction model or filtered out. The emerging technology may also be able to in real-time or temporally discriminate plaque size and rate of growth, potentially revealing information in regard to a quality control monitoring, outlier detection, and root cause analysis investigations for a number of quality parameters relating to the virus sample tested including aggregation status of the original sample, viral potency, and variation within the population due to mutations. The multivariate data analysis based on the extracted or generated image feature sets or other related metadata may also function as a discovery tool for the identification of other undetermined virus/cell interaction features through the detection of other variations in plaque morphology. Such variations in plaque morphology may not be distinguishable by current methods but are revealed by the multi-parametric analysis and throughput enabled by the combination of automation machine learning, multivariate data analysis and live cell imaging, which are aspects of this disclosure.

As another benefit of the methods of this disclosure, the plaque size required for detection is reduced, therefore effectively allowing more plaques per unit area/field of view without risk of overlap. As such, this effectively decreases the dilution series that is required, lessening a significant labor burden. The other alternative here is that one can decrease the area needed to examine plaques from an appropriate test dilution and potentially move to smaller formats, with higher throughput.

Specialist media may be added to the culture plate, e.g. detection aids specific to the virus or containing agents that aid imaging generally and in a machine learning specific manner. The latter might have more general application. Such media could, for example, have reagents that react to the release of cell contents or that aggregate on viral antigens (e.g. antibodies) and either carry detectable markers or through their aggregation form detectable structures. Such reagents might be either fluorogenic dyes or dyes with higher quantum yields at lower concentrations in the medium or other reagents such as molecular tags designed for detection through specific imaging methods, e.g. Raman spectroscopy. Reagents could be used for early detection of specific cytopathic effects such as, but not limited to, remodeling of the cytoskeleton, live/dead detection via membrane integrity changes, activation of apoptosis and autophagy pathways, cell cycle, and oxidative stress.

Antibody and other labels can facilitate the generation of mathematical algorithms up-front by adding classical identification for the training models in machine learning or Al that would be derived from these images, i.e. they can tell the person doing the modeling where the action is, where to look. These reagents can also be used for model confirmation. This is true in cases were the gross effects of virus infection are less obvious in a heterogeneous or raw preparation of virus where cytopathic effects might be more subtle. This may result with some viruses that may not be lytic or may not be lytic within the same time period as the more virulent viruses in a preparation.

Such binding molecules can provide additional information through the addition of information on the chemical and molecular composition of an area to the physical structural information detectable.

As stated previously, the present methods now allow for the essentially contemporaneous determination of (1) total particle count (by means of an assay of the sample in a viral particle counting instrument such as Sartorius' Virus Counter^{®} ) and (2) infectious particle count, via the viral plaque assay of this disclosure. Both assays can be conducted in parallel, at the same time, in separate instruments or platforms.

Finally, these techniques may also be applied to new chemical and biological entity potency assays developed and used with adhered cells outside of virology. These would include, but not be limited to, neutralization, cell proliferation, cell death (apoptosis), cytokine release, modulation of cell signaling, modulation of inflammatory response, receptor binding/activation, ligand binding, and calcium flux.

The applications for this invention are quite broad. The majority of the virus quantification market across basic research, development and manufacturing considers the plaque assay to be the standard assay. The applications include, but are not limited to:
1. basic research (academic or industrial),
2. assay development,
3. process development and production, including gene therapy, protein manufacturing via expression with baculovirus, and viral vaccines,
4. antiviral screening and development,
5. manufacturing Quality Control (QC),
6. Conversion Rate Optimization (CRO) testing,
7. viral stock establishment,
8. virus removal and/or inactivation, and
9. Good Manufacturing Process (GMP) validation and non-GMP studies, and
10. potency assays for new chemical or biological entities.

The appended claims define the scope of the disclosed inventions. The above detailed description describes various features and functions of the disclosed systems, devices, and methods with reference to the accompanying figures. In the figures, similar symbols typically identify similar components, unless context indicates otherwise. The illustrative embodiments described in the detailed description, figures, and claims are not meant to be limiting. Other embodiments can be utilized, and other changes can be made, without departing from the scope of the subject matter presented herein. It will be readily understood that the aspects of the present disclosure, as generally described herein, and illustrated in the figures, can be arranged, substituted, combined, separated, and designed in a wide variety of different configurations, all of which are explicitly contemplated herein.

With respect to any or all of the message flow diagrams, scenarios, and flowcharts in the figures and as discussed herein, each step, block and/or communication may represent a processing of information and/or a transmission of information in accordance with example embodiments. In alternative embodiments, for example, functions described as steps, blocks, transmissions, communications, requests, responses, and/or messages may be executed out of order from that shown or discussed, including in substantially concurrent or in reverse order, depending on the functionality involved. Further, more or fewer steps, blocks and/or functions may be used with any of the message flow diagrams, scenarios, and flow charts discussed herein, and these message flow diagrams, scenarios, and flow charts may be combined with one another, in part or in whole.

A step or block that represents a processing of information may correspond to circuitry that can be configured to perform the specific logical functions of a herein-described method or technique. Alternatively, or additionally, a step or block that represents a processing of information may correspond to a module, a segment, or a portion of program code (including related data). The program code may include one or more instructions executable by a processor for implementing specific logical functions or actions in the method or technique. The program code and/or related data may be stored on any type of computer-readable medium, such as a storage device, including a disk drive, a hard drive, or other storage media.

The computer-readable medium may also include non-transitory computer-readable media such as computer-readable media that stores data for short periods of time like register memory, processor cache, and/or random access memory (RAM). The computer-readable media may also include non-transitory computer-readable media that stores program code and/or data for longer periods of time, such as secondary or persistent long term storage, like read only memory (ROM), optical or magnetic disks, and/or compact-disc read only memory (CD-ROM), for example. The computer-readable media may also be any other volatile or nonvolatile storage systems. A computer-readable medium may be considered a computer-readable storage medium, for example, or a tangible storage device.

Moreover, a step or block that represents one or more information transmissions may correspond to information transmissions between software and/or hardware modules in the same physical device. However, other information transmissions may be between software modules and/or hardware modules in different physical devices.

While various aspects and embodiments have been disclosed for purposes illustration and not limitation, it will be apparent to those skilled in the art that variation from the specifics of this disclosure are possible without departure from the scope of the invention, which is defined by the appended claims.

## Claims

1. A method for training a machine learning model to predict a virus titer from a sequence of images of a cell culture containing a virus population, comprising the steps of:
(1) obtaining a training set in a form of a plurality of images of virus-treated cell cultures from a plurality of experiments at a plurality of time points from a start time t₀ to a final time t_{final}, wherein the training set images comprise fluorescence images of the cell culture labelled with a fluorescent marker, wherein the fluorescent marker is a fluorescent antibody binding to virus specific protein epitopes;
(2) for each experiment, recording at least one numeric virus titer readout of the virus-treated cell culture at the final time t_{final};
(3) processing the images in the training set to acquire a numeric representation of each image in the sequence captured at a time before t_{final}; and
(4) training one or more machine learning models to make a prediction of a final virus titer on the training set numeric representations,
wherein the final virus titer ground truth is represented by the corresponding numeric virus titer readout.

2. The method of claim 1, wherein the virus titer readout comprises (1) a number of infective particles or number of infective particles per unit volume, (2) a readout of a Tissue Culture Infective Dose 50 % Assay, or (3) the readout from a focus-forming assay.

3. The method of any of claims 1-2, wherein the training set images comprise label-free light microscopy images.

4. The method of any of claims 1-3, wherein processing step (3) comprises passing the microscopic images through a convolutional neural network (CNN) to thereby acquire an intermediate data representation of the microscopic images.

5. The method of any of claims 1-4, wherein the processing step (3) comprises the steps of: a) segmenting individual cells from the microscopic images, b) calculating a cell-by-cell numeric description of each cell, and c) aggregating the numeric descriptions over all cells.

6. The method of any of claims 1-5, wherein the machine learning model comprises one of a) a partial least squares linear model, b) an artificial neural network, c) a Gaussian process regression, and d) a neural ordinary differential equation model.

7. The method of any of claims 1-6, wherein there are at least two time points in step 1) and wherein the period between the time points is less than or equal to 60 minutes.

8. A method for predicting a virus titer of a cell culture to which a virus sample of unknown titer has been added, comprising the steps of:
a) obtaining a time sequence of microscopic images of the cell culture;
b) supplying a numeric representation of the time sequence of microscopic images obtained in step a) to one or more machine learning models trained in accordance with any one of claims 1-7, and
c) making a prediction with the one or more trained machine learning models of the virus titer.

9. The method of claim 8, wherein the cell culture further comprises specialist media aiding in imaging of the cell culture.

10. An analytical instrument, comprising
a system configured to hold one or more plates containing a cell culture and a virus sample,
an integrated microscopic imaging system, and
a machine learning model trained to make a prediction of a virus titer in the cell culture from one or more images in a time sequence of images of the cell culture obtained by the microscopic imaging system, wherein the prediction is made before the viral infection of the cell culture has proceeded to term, wherein the images comprise fluorescence images of the cell culture labelled with a fluorescent marker, wherein the fluorescent marker is a fluorescent antibody binding to virus specific protein epitopes.

11. The analytical instrument of claim 10, wherein the instrument is further configured with a processing unit executing a training module, the training module providing set-up instructions for facilitating a user of the instrument conducting a training method with the instrument comprising the steps of:
(1) obtaining a training set in the form of a a plurality of images of virus-treated cell cultures from a plurality of experiments at a set of time points from a start time t₀ to a final time t_{final},
(2) for each experiment, recording at least one numeric virus titer readout of the virus-treated cell culture at time t_{final},
(3) processing all microscopic images in the training set to acquire a numeric representation of each image; and
(4) training one or more machine learning models to make a prediction of a final virus titer on the training set numeric representations, wherein the training comprises minimizing the error between the model prediction of a final virus titer and a ground truth.

12. The analytical instrument of claim 11, wherein the processing step (3) comprises the steps of: a) segmenting individual cells from the microscopic image, b) calculating a cell-by-cell numeric description of each cell, and c) aggregating the numeric descriptions over all cells.

13. The analytical instrument of claim 11, wherein the machine learning model comprises one of a) a partial least squares linear model, b) an artificial neural network, c) a Gaussian process regression, and d) a neural ordinary differential equation model.

14. The analytical instrument of claim 11, wherein the processing step (3) further comprises a step of either (1) filtering out cells not infected by the virus, (2) filtering out dead cells, or (3) filtering out dead cells that did not die from a virus infection.

## Patentansprüche

1. Verfahren zum Trainieren eines maschinellen Lernmodells zur Vorhersage eines Virustiters aus einer Bildsequenz einer Zellkultur, die eine Viruspopulation enthält, umfassend die Schritte:
(1) Erlangen eines Trainingssatzes in Form einer Vielzahl von Bildern von virusbehandelten Zellkulturen aus einer Vielzahl von Experimenten zu einer Vielzahl von Zeitpunkten von einem Startzeitpunkt t₀ bis zu einem Endzeitpunkt t_{final}, wobei die Bilder des Trainingssatzes Fluoreszenzbilder der Zellkultur umfassen, die mit einem Fluoreszenzmarker markiert sind, wobei der Fluoreszenzmarker ein fluoreszierender Antikörper ist, der an virusspezifische Proteinepitope bindet;
(2) Aufzeichnen, für jedes Experiment, mindestens einer numerischen Virustiterablesung der virusbehandelten Zellkultur zum Endzeitpunkt t_{final};
(3) Verarbeiten der Bilder in dem Trainingssatz, um eine numerische Darstellung jedes Bildes in der Sequenz zu erhalten, das zu einem Zeitpunkt vor t_{final} aufgenommen wurde; und
(4) Trainieren eines oder mehrerer maschineller Lernmodelle, um eine Vorhersage des endgültigen Virustiters auf der Grundlage der numerischen Darstellungen des Trainingssatzes zu treffen,
wobei die endgültige Virustiter-Grundwahrheit durch die entsprechende numerische Virustiterablesung dargestellt wird.

2. Verfahren nach Anspruch 1, wobei die Virustiterablesung (1) eine Anzahl infektiöser Partikel oder eine Anzahl infektiöser Partikel pro Volumeneinheit, (2) eine Ablesung eines Gewebekultur-Infektionsdosis-50%-Assays oder (3) die Ablesung eines fokusbildenden Assays umfasst.

3. Verfahren nach einem der Ansprüche 1 bis 2, wobei die Bilder des Trainingssatzes markierungsfreie lichtmikroskopische Bilder umfassen.

4. Verfahren nach einem der Ansprüche 1 bis 3, wobei der Verarbeitungsschritt (3) das Durchlaufen der mikroskopischen Bilder durch ein neuronales Faltungsnetzwerk (CNN) umfasst, um dadurch eine Zwischendatendarstellung der mikroskopischen Bilder zu erhalten.

5. Verfahren nach einem der Ansprüche 1 bis 4, wobei der Verarbeitungsschritt (3) die folgenden Schritte umfasst: a) Segmentieren einzelner Zellen aus den mikroskopischen Bildern, b) Berechnen einer zellenweisen numerischen Beschreibung jeder Zelle, und c) Aggregieren der numerischen Beschreibungen über alle Zellen.

6. Verfahren nach einem der Ansprüche 1 bis 5, wobei das maschinelle Lernmodell eines der folgenden Modelle umfasst: a) ein lineares Modell der partiellen kleinsten Quadrate, b) ein künstliches neuronales Netzwerk, c) eine Gaußsche Prozessregression und d) ein neuronales gewöhnliches Differentialgleichungsmodell.

7. Verfahren nach einem der Ansprüche 1 bis 6, wobei es in Schritt 1) mindestens zwei Zeitpunkte gibt und wobei der Zeitraum zwischen den Zeitpunkten kleiner oder gleich 60 Minuten ist.

8. Verfahren zur Vorhersage eines Virustiters einer Zellkultur, der eine Virusprobe mit unbekanntem Titer zugesetzt wurde, umfassend die folgenden Schritte:
a) Erlangen einer zeitliche Abfolge von mikroskopischen Bildern der Zellkultur;
b) Bereitstellen einer numerischen Darstellung der in Schritt a) erlangten zeitlichen Abfolge von mikroskopischen Bildern für ein oder mehrere maschinelle Lernmodelle, die gemäß einem der Ansprüche 1 bis 7 trainiert wurden, und
c) Treffen einer Vorhersage des Virustiters mit einem oder mehreren trainierten maschinellen Lernmodellen.

9. Verfahren nach Anspruch 8, wobei die Zellkultur ferner Spezialmedien umfasst, die bei der Abbildung der Zellkultur helfen.

10. Analyseinstrument, umfassend
ein System, das konfiguriert ist, um eine oder mehrere Platten aufzunehmen, die eine Zellkultur und eine Virusprobe enthalten,
ein integriertes mikroskopisches Abbildungssystem, und
ein maschinelles Lernmodell, das trainiert wurde, um eine Vorhersage eines Virustiters in der Zellkultur aus einem oder mehreren Bildern in einer zeitlichen Abfolge von Bildern der Zellkultur, die durch das mikroskopische Abbildungssystem erhalten wurden, zu treffen, wobei die Vorhersage getroffen wird, bevor die virale Infektion der Zellkultur bis zum Ende fortgeschritten ist, wobei die Bilder Fluoreszenzbilder der Zellkultur umfassen, die mit einem Fluoreszenzmarker markiert sind, wobei der Fluoreszenzmarker ein fluoreszierender Antikörper ist, der an virusspezifische Proteinepitope bindet.

11. Analyseinstrument nach Anspruch 10, wobei das Instrument ferner mit einer Verarbeitungseinheit konfiguriert ist, die ein Trainingsmodul ausführt, wobei das Trainingsmodul Einrichtungsanweisungen bereitstellt, um einem Benutzer des Instruments die Durchführung eines Trainingsverfahrens mit dem Instrument zu erleichtern, das die folgenden Schritte umfasst:
(1) Erlangen eines Trainingssatzes in Form einer Vielzahl von Bildern von virusbehandelten Zellkulturen aus einer Vielzahl von Experimenten zu einer Vielzahl von Zeitpunkten von einem Startzeitpunkt t₀ bis zu einem Endzeitpunkt t_{final},
(2) Aufzeichnen, für jedes Experiment, mindestens einer numerischen Virustiterablesung der virusbehandelten Zellkultur zum Zeitpunkt t_{final},
(3) Verarbeiten aller mikroskopischen Bilder in dem Trainingssatz, um eine numerische Darstellung jedes Bildes zu erhalten; und
(4) Trainieren eines oder mehrerer maschineller Lernmodelle, um eine Vorhersage eines endgültigen Virustiters auf der Grundlage der numerischen Darstellungen des Trainingssatzes zu treffen, wobei das Trainieren das Minimieren des Fehlers zwischen der Modellvorhersage eines endgültigen Virustiters und einer Grundwahrheit umfasst.

12. Analyseinstrument nach Anspruch 11, wobei der Verarbeitungsschritt (3) die folgenden Schritte umfasst: a) Segmentieren einzelner Zellen aus dem mikroskopischen Bild, b) Berechnen einer zellenweisen numerischen Beschreibung jeder Zelle, und c) Aggregieren der numerischen Beschreibungen über alle Zellen.

13. Analyseinstrument nach Anspruch 11, wobei das maschinelle Lernmodell eines der folgenden Modelle umfasst: a) ein lineares Modell der partiellen kleinsten Quadrate, b) ein künstliches neuronales Netzwerk, c) eine Gaußsche Prozessregression und d) ein neuronales gewöhnliches Differentialgleichungsmodell.

14. Analyseinstrument nach Anspruch 11, wobei der Verarbeitungsschritt (3) ferner einen Schritt umfasst, bei dem entweder (1) Zellen herausgefiltert werden, die nicht von dem Virus infiziert sind, (2) tote Zellen herausgefiltert werden oder (3) tote Zellen herausgefiltert werden, die nicht an einer Virusinfektion gestorben sind.

## Revendications

1. Procédé pour entraîner un modèle d'apprentissage machine afin de prédire un titre viral à partir d'une séquence d'images d'une culture cellulaire comprenant une population de virus, comprenant les étapes de :
(1) obtention d'un jeu d'entraînement sous la forme d'une pluralité d'images de cultures cellulaires traitées par un virus issues d'une pluralité d'expériences à une pluralité de points de temps allant d'un moment de départ t₀ à un moment final t_{final}, dans laquelle les images du jeu d'entraînement comprennent des images de fluorescence de la culture cellulaire marquée avec un marqueur fluorescent, dans laquelle le marqueur fluorescent est un anticorps fluorescent se liant à des épitopes protéiques spécifiques d'un virus ;
(2) pour chaque expérience, enregistrement d'au moins une lecture de titre viral numérique de la culture cellulaire traitée par un virus au moment final t_{final} ;
(3) traitement des images dans le jeu d'entraînement pour acquérir une représentation numérique de chaque image dans la séquence capturée à un moment avant t_{final} ; et
(4) entraînement d'un ou plusieurs modèles d'apprentissage machine pour réaliser une prédiction d'un titre viral final sur les représentations numériques du jeu d'entraînement ;
dans lequel la vérité terrain du titre viral final est représentée par la lecture du titre viral numérique correspondant.

2. Procédé selon la revendication 1, dans lequel la lecture de titre viral comprend (1) le nombre de particules infectieuses ou le nombre de particules infectieuses par unité de volume, (2), la lecture d'un dosage de la dose infectieuse en culture tissulaire à 50 %, ou (3) la lecture provenant d'un dosage de formation de foyers.

3. Procédé selon l'une quelconque des revendications 1 et 2, dans lequel les images du jeu d'entraînement comprennent des images de microscope optique sans marqueur.

4. Procédé selon l'une quelconque des revendications 1 à 3, dans lequel l'étape de traitement (3) comprend le passage des images de microscope dans un réseau neuronal convolutif (CNN) afin d'acquérir ainsi une représentation de données intermédiaires des images de microscope.

5. Procédé selon l'une quelconque des revendications 1 à 4, dans lequel l'étape de traitement (3) comprend les étapes de : a) segmentation de cellules individuelles à partir des images de microscope, b) calcul d'une description numérique cellule par cellule de chaque cellule, et c) agrégation des descriptions numériques sur toutes les cellules.

6. Procédé selon l'une quelconque des revendications 1 à 5, dans lequel le modèle d'apprentissage machine comprend l'un parmi a) un modèle linéaire des moindres carrés partiels, b) un réseau neuronal artificiel, c) une régression par processus gaussien, et d) un modèle neural d'équation différentielle ordinaire.

7. Procédé selon l'une quelconque des revendications 1 à 6, dans lequel il y a au moins deux points de temps dans l'étape 1), et dans lequel la période entre les points de temps est inférieure ou égale à 60 minutes.

8. Procédé pour prédire un titre viral d'une culture cellulaire à laquelle un échantillon viral de titre inconnu a été ajouté, comprenant les étapes de :
a) obtention d'une séquence temporelle d'images de microscope de la culture cellulaire ;
b) fourniture d'une représentation numérique de la séquence temporelle d'images de microscope obtenue dans l'étape a) à un ou plusieurs modèles d'apprentissage machine entraînés selon l'une quelconque des revendications 1 à 7, et
c) réalisation d'une prédiction avec le ou les modèles d'apprentissage machine entraînés du titre viral.

9. Procédé selon la revendication 8, dans lequel la culture cellulaire comprend en outre un milieu spécialisé facilitant la prise d'images de la culture cellulaire.

10. Instrument analytique comprenant :
un système configuré pour retenir une ou plusieurs plaques contenant une culture cellulaire et un échantillon viral,
un système d'imagerie au microscope intégré, et
un modèle d'apprentissage machine entraîné pour réaliser une prédiction d'un titre viral dans la culture cellulaire à partir d'une ou plusieurs images dans une séquence temporelle d'images de la culture cellulaire obtenue par le système d'imagerie au microscope, dans lequel la prédiction est réalisée avant que l'infection virale de la culture cellulaire se soit déroulée jusqu'à son terme, dans lequel les images comprennent des images de fluorescence de la culture cellulaire marquée avec un marqueur fluorescent, dans lequel le marqueur fluorescent est un anticorps fluorescent se liant à des épitopes protéiques spécifiques d'un virus.

11. Instrument analytique selon la revendication 10, lequel instrument est en outre configuré avec une unité de traitement exécutant un module d'entraînement, le module d'entraînement fournissant des instructions de configuration pour aider un utilisateur de l'instrument effectuant un procédé d'apprentissage avec l'instrument, comprenant les étapes de :
(1) obtention d'un jeu d'entraînement sous la forme d'une pluralité d'images de cultures cellulaires traitées par un virus issues d'une pluralité d'expériences à une pluralité de points de temps allant d'un moment de départ t₀ à un moment final t_{final},
(2) pour chaque expérience, enregistrement d'au moins une lecture de titre viral numérique de la culture cellulaire traitée par un virus au moment t_{final} ;
(3) traitement de toutes les images de microscope dans le jeu d'entraînement pour acquérir une représentation numérique de chaque image ; et
(4) entraînement d'un ou plusieurs modèles d'apprentissage machine pour réaliser une prédiction d'un titre viral final sur les représentations numériques du jeu d'entraînement, lequel entraînement comprend une minimisation de l'erreur entre la prédiction modélisée du titre viral final et la vérité terrain.

12. Instrument analytique selon la revendication 11, dans lequel l'étape de traitement (3) comprend les étapes de : a) segmentation de cellules individuelles à partir des images de microscope, b) calcul d'une description numérique cellule par cellule de chaque cellule, et c) agrégation des descriptions numériques sur toutes les cellules.

13. Instrument analytique selon la revendication 11, dans lequel le modèle d'apprentissage machine comprend l'un parmi a) un modèle linéaire des moindres carrés partiels, b) un réseau neuronal artificiel, c) une régression par processus gaussien, et d) un modèle neural d'équation différentielle ordinaire.

14. Instrument analytique selon la revendication 11, dans lequel l'étape de traitement (3) comprend en outre une étape de soit (1) élimination par filtration des cellules non infectées par le virus, soit (2) élimination par filtration des cellules mortes, soit (3) élimination par filtration des cellules mortes qui ne sont pas mortes du fait d'une infection virale.
